(19) Europäisches Patentamt
European Patent Office
Office européen des brevets

(11) Numéro de publication: **0 007 255**
**B1**

(12) **FASCICULE DE BREVET EUROPEEN**

(45) Date de publication du fascicule du brevet: **06.07.83**

(21) Numéro de dépôt: **79400322.8**

(22) Date de dépôt: **22.05.79**

(51) Int. Cl.³: **C 07 C 121/75,** C 07 B 19/00, A 01 N 53/00

(54) **Esters d'acides cyclopropane carboxyliques substitués d'alcool alpha-cyané, leur procédé de préparation, les compositions pesticides les renfermant ainsi que leur application à titre de médicaments à usage vétérinaire.**

(30) Priorité: **06.06.78 FR 7816858**

(43) Date de publication de la demande:
**23.01.80 Bulletin 80/2**

(45) Mention de la délivrance du brevet:
**06.07.83 Bulletin 83/27**

(84) Etats contractants désignés:
**BE CH DE FR GB IT NL**

(56) Documents cités:
CH - A - 491 851
DE - A - 2 718 039
FR - A - 2 143 820
FR - A - 2 232 273
FR - A - 2 240 914

(73) Titulaire: **ROUSSEL-UCLAF**
**102, route de Noisy Boîte postale no.9**
**F-93230 Romainville (FR)**

(72) Inventeur: **Martel, Jacques**
**15, rue Douvillez**
**F-93140 Bondy F (FR)**
Inventeur: **Tessier, Jean**
**30, rue Jean Moulin**
**F-94300 Vincennes (FR)**

(74) Mandataire: **Niel, Michel et al,**
**boîte postale no 9 102, route de Noisy**
**F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 007 255 B1

Esters d'acides cyclopropane carboxyliques substitués d'alcool α-cyané, leur procédé de préparation, les compositions pesticides les renfermant ainsi que leur application à titre de médicaments à usage vétérinaire.

On connaissait déjà de nombreux esters de l'acide cyclopropane carboxylique et de l'alcool (RS) α-cyano 3-phénoxy benzylique, présentant des propriétés insecticides et acaricides, pour lutter contre les acariens parasites des végétaux, (voir à ce sujet, les brevets français 2.232.273, 2.143.820, 2.718.039 et 2.240.914.

On vient de découvrir de nouveaux esters dérivés de l'acide cyclopropane carboxylique et de l'alcool (S) α-cyano 3-phénoxy benzylique présentant des propriétés nématicides et acaricides pour lutter contre les acariens parasites des animaux tout à fait remarquables. On vient également de découvrir que certains esters connus de l'alcool (RS) α-cyano 3-phénoxy benzylique présentaient des propriétés nématicides et acaracides destinées à la lutte contre les acariens parasites des animaux.

L'invention a plus précisément pour object, les composés de formule ($I_A$):

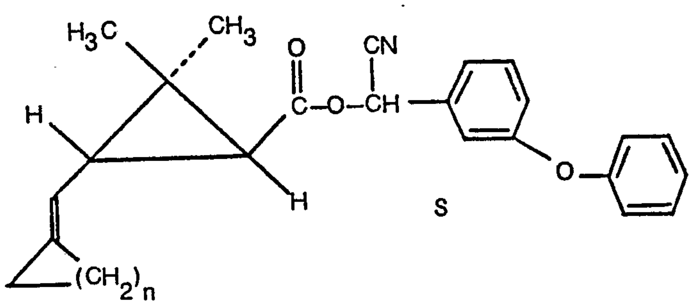

($I_A$)

dans laquelle n est un entier égal à 1, 2 ou 3, la configuration de la copule acide est (1R, trans) ou (1R, cis) et celle de la copule alcoolique est (S).

L'invention a notamment pour objet:

— le 1R, trans 2,2-diméthyl 3-(cyclopentylidène méthyl)cyclopropane-1-carboxylate de (S) α-cyano 3-phénoxy benzyle,

— le 1R, cis 2,2-diméthyl 3-(cyclopentylidène méthyl)cyclopropane-1-carboxylate de (S) α-cyano 3-phénoxy benzyle,

— le 1R, cis 2,2-diméthyl 3-(cyclobutylidène méthyl)cyclopropane-1-carboxylate de (S) α-cyano 3-phénoxy benzyle,

— le 1R, trans 2,2-diméthyl 3-(cyclobutylidène méthyl)cyclopropane-1-carboxylate de (S) α-cyano 3-phénoxy benzyle,

— le 1R, cis 2,2-diméthyl 3-(cyclopropylidène méthyl)cyclopropane-1-carboxylate de (S) α-cyano 3-phénoxy benzyle,

— le 1R, trans 2,2-diméthyl 3-(cyclopropylidène méthyl)cyclopropane-1-carboxylate de (S) α-cyano 3-phénoxy benzyle.

Les composés de formule ($I_A$) comportent en position 1 et en position 3 du cycle cyclopropanique, des carbones asymétriques, le carbone en position 1 étant de configuration (R) et les groupements fixés en positions 1 et 3 de ce cycle sont cis ou trans.

Pour des raisons de commodité, ce genre de structure est actuellement désigné, d'une façon abrégée, par "(1R, cis)" ou "(1R, trans)".

L'invention a également pour objet un procédé de préparation des composés de formule ($I_A$), caractérisé en ce que l'on fait réagir un acide de formule (II) de structure 1R, cis ou 1R, trans:

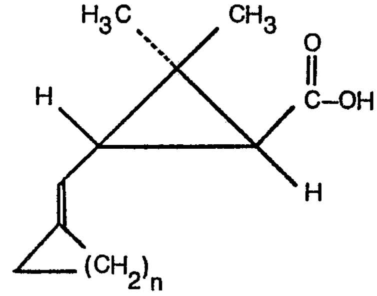

(II)

dans lequel n est défini comme précédemment ou l'un de ses dérivés fonctionnels, avec l'alcool (S) $\alpha$-cyano 3-phénoxy benzylique ou avec l'alcool (RS) $\alpha$-cyano 3-phénoxy benzylique, pour obtenir l'ester correspondant de (S) ou de (RS) $\alpha$-cyano 3-phénoxy benzyle, et soumet, le cas échéant, l'ester de (RS) $\alpha$-cyano 3-phénoxy benzyle à l'action d'un agent basique au sein d'un solvant dans lequel l'ester correspondant d'alcool de configuration (S) est insoluble et dans lequel l'ester correspondant d'alcool de configuration (R) est soluble, pour obtenir l'ester de (S) $\alpha$-cyano 3-phénoxy benzyle.

Dans des conditions préférentielles de mise en oeuvre de ce procédé, on utilise au départ un halogénure de l'acide de formule (II), plus particulièrement le chlorure, et l'on opère en présence d'une base, plus particulièrement une base tertiaire telle que la pyridine ou la triéthylamine, au sein d'un solvant organique, tel que le benzène.

L'invention a plus particulièrement pour objet un procédé selon ce qui précède, caractérisé, en ce que l'on soumet l'ester obtenu de (RS) $\alpha$-cyano 3-phénoxy benzyle à l'action d'un agent basique, au sein d'un solvant dans lequel l'ester correspondant d'alcool de configuration (S) est insoluble et dans lequel l'ester correspondant d'alcool de configuration (R) est soluble, pour obtenir l'ester de (S) $\alpha$-cyano 3-phénoxy benzyle.

Les esters de (S) $\alpha$-cyano 3-phénoxy benzyle de formule ($I_A$) peuvent ainsi, selon l'invention, être obtenus soit par estérification de l'alcool (S) $\alpha$-cyano 3-phénoxy benzylique par un acide de formule (II) ou un dérivé fonctionnel de cet acide, soit, par transformation de l'ester d'alcool (RS) en ester d'alcool (S) par action d'un agent basique, au sein d'un solvant dans lequel l'ester d'alcool (S) est insoluble et dans lequel l'ester correspondant d'alcool (R) est soluble, puis isolement par un moyen physique de l'ester d'alcool (S) ainsi insolubilisé.

Ce procédé de transformation d'ester d'alcool RS en ester d'alcool (S) est particulièrement remarquable. Il a été décrit d'une façon générale dans la demande de brevet français de la société demanderesse déposée le 23 Avril 1976 sous le n° 76 12094 et intitulée "Procédé de transformation d'un ester d'acide chiral d'alcool secondaire $\alpha$-cyané optiquement actif de structure (R) en ester d'acide chiral d'alcool secondaire $\alpha$-cyané de structure (S)".

Selon des modes d'exécution préférés du procédé de l'invention, l'agent basique utilisé pour la transformation de l'ester d'alcool (RS) en ester d'alcool (S) est choisi dans le groupe constitué par l'ammoniaque, la triéthylamine, la diéthylamine, la morpholine, la pyrrolidine, la pipéridine et utilisés en quantité catalytique, la soude, la potasse, les alcoolates alcalins, les amidures alcalins, les hydrures alcalins, et le solvant ou le mélange de solvants utilisés pour insolubiliser l'ester d'alcool (S) est choisi dans le groupe constitué par l'acétonitrile, les alcanols et les mélanges d'alcanol et d'éther de pétrole, notamment les mélanges d'alcanol et de pentane, d'hexane ou d'heptane.

Par exemple, selon un mode opératoire commode du procédé de l'invention, on utilise l'ammoniaque pour transformer l'ester d'alcool (RS) en ester d'alcool (S), en opérant au sein de l'isopropanol.

Les composés de formule I:

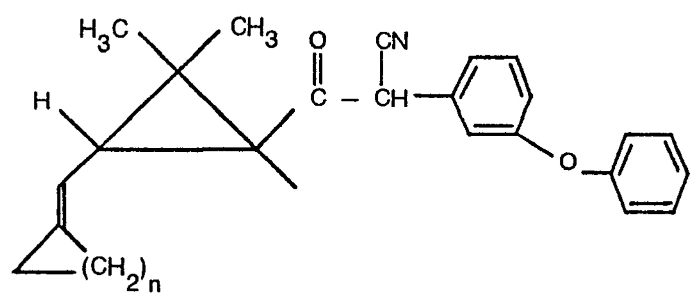

dans laquelle n est un entier égal à 1, 2 ou 3, la configuration de la copule acide est (1R, trans) ou (1R, cis) et celle de la copule alcoolique est RS ou S, sont doués d'intéressantes propriétés nématicides et peuvent être notamment utilisés dans le domain agricole, dans la lutte contre les nématodes parasites des végétaux cultivés.

L'activité nématicide des composés I peut être mise en évidence, notamment par des tests sur Ditylenchus myceliophagus. Ces tests sont décrits plus loin dans la partie expérimentale.

Les composés de formule (I) sont également doués d'intéressantes propriétés acaricides vis-à-vis des acariens parasites des animaux et peuvent ainsi être utilisés dans la lutte contre les ixodidés et les sarcoptidés parasites des animaux, notamment contre toutes sortes de gales, telles que la gale sarcoptique, la gale psoroptique et la gale chorioptique, ou contre toutes sortes de tiques, telles que l'espèce Boophilus, l'espèce Hyalomnia, l'espèce Amblyoma et l'espèce Rhipicephalus. Des tests sont décrits ci-après dans la partie expérimentale.

L'invention a aussi pour objet les compositions nématicides caractérisées en ce qu'elles contiennent, comme principe actif un au moins des composés de formule I.

Les compositions nématicides de l'invention peuvent se présenter notamment sous forme de poudre, granulés, suspensions, émulsions, solutions.

Dans ces compositions, le ou les principes actifs peuvent être additionnés éventuellement d'un ou plusieurs autres agents pesticides.

Pour la lutte contre les nématodes parasites des cultures, on utilise, de préférence, des liquides pour traitement des sols, contenant de 30 à 500 g/l de principe actif. Les composés nématicides, selon l'invention, sont utilisés de préférence à des doses comprises entre 3 et 300 g de matière active à l'hectare.

L'invention a aussi pour objet l'application des produits de formule (I), à la lutte contre les acariens parasites des animaux, et notamment l'application des produits de formule (I), à titre de médicaments vétérinaires, employés à cet effet.

L'invention a enfin pour objet les compositions renfermant, à titre de principe actif, l'un au moins des produits de formule (I) et destinées à la lutte contre les acariens parasites des animaux et notamment les compositions pharmaceutiques vétérinaires renfermant, à titre de principe actif, l'un au moins des produits de formule (I), destinées à cet effet.

Les compositions selon l'invention peuvent être utilisées par voie externe, mais également par voie parentérale ou digestive. Elles peuvent être avantageusement additionnées d'un agent synergisant des pyréthrinoïdes.

Les compositions destinées à la lutte contre les acariens parasites des animaux, telles que définies ci-dessus, peuvent être préparées selon les techniques usuelles.

Pour l'application locale, par exemple, on utilise des solutions émulsionnables dans l'eau, à diluer elles-mêmes dans l'eau au moment de l'emploi; elles renferment, en général, de 1/500 à 1/5 en poids de principe actif et, de préférence, de 1/100 à 1/10. Ces solutions renferment le plus souvent une grande quantité d'agents synergisants des pyréthrinoïdes, tels qu'indiqués précédemment elles peuvent renfermer, par exemple, une quantité pondérale de butoxyde de pipéronyle comprise entre 2 et 20 fois celle du principe actif et, de préférence, entre 5 et 12 fois.

Ces solutions renferment, en général, également des émulsifiants comme les "Tween" et les "Span"; on utilise, de préférence, des agents émulsifiants non ioniques comme le Polysorbate 80 ou le Triton X 100. Ces agents émulsifiants ont pour rôle de favoriser le mouillage et la pénétration du principe actif dans les lésions de la peau. On utilise, de préférence, une quantité pondérale d'agents émulsifiants comprise entre 2 et 20 fois celle du principe actif, de préférence, entre 5 et 10 fois.

Ces solutions peuvent renfermer aussi un agent antioxydant soluble dans les solvants organiques comme l'acétate de Tocophérol.

Le principe actif et les divers agents, par exemple, les agents synergisants, émulsifiants et antioxydants sont en général en solution dans un alcool comme l'alcool éthylique ou bien dans un mélange d'alcool éthylique, d'alcool et isopropylique ou bien dans un mélange d'alcool éthylique, d'alcool isopropylique ou d'acétate d'éthyle.

La posologie, pour l'usage externe, varie avec les animaux à traiter et les parasites qu'ils hébergent. Pour le (1R, trans) 2,2-diméthyl 3-(cyclopentylidène méthyl) cyclopropane-1-carboxylate de (RS) $\alpha$-cyano 3-phénoxy benzyle, par exemple, on utilisera avantageusement des solutions au 1/1000.

Pour l'usage par voie sous-cutanée ou intramusculaire, on utilise, par exemple, les composés I en solution dans un excipient convenant à cet usage, tel qu'un mélange de benzoate de benzyle et d'huile d'arachide. Les solutions pour injections peuvent également contenir de l'acétate d'$\alpha$-tocophérol et du butoxyde de pipéronyle.

Pour l'usage par voie orale, on peut utiliser des capsules.

Il peut être commode, pour l'usage vétérinaire, d'utiliser les composés selon l'invention, en mélange avec les aliments composés équilibrés pour animaux.

On pourra, par exemple, employer des aliments composés pour animaux qui renferment de 0,006 à 0,8% de matière active. Ces aliments pourront également, selon l'invention, renfermer un agent synergisant.

L'alcool (S) $\alpha$-cyano 3-phénoxy benzylique est décrit dans la demande de brevet français déposée par la société demanderesse le 31 Janvier 1978 sous le n° 78 02621 et intitulée "Alcool benzylique substitué optiquement actif et son procédé de préparation".

Les exemples suivants illustrent l'invention sans la limiter.

### Exemple 1:

*(1R trans) 2,2-diméthyl 3-(cyclopentylidène méthyl) cyclopropane-1-carboxylate de (R,S) $\alpha$-cyano 3-phénoxy benzyle*

Dans une solution de 2,8 g d'alcool (R S) $\alpha$-cyano 3-phénoxy benzylique dans 10 ml de benzène anydre, on introduit 1.5 ml de pyridine. On maintient la température vers 20°C et ajout 3,17 g de chlorure d'acide 1R, trans 2,2-diméthyl 3-(cyclopentylidène méthyl) cyclopropane-1-carboxylique en solution dans du benzène. Après 20 heures d'agitation, on ajoute 20 ml d'une solution d'acide chlorhydrique dilué, puis extrait au chlorure de méthylène. On isole les phases organiques, les lave à l'eau, les sèche, et concentre à sec. Après purification par chromatographie sur silice (éluant: cyclohexane acétate d'éthyle 9—1) on obtient 3,5 g de produit attendu.

$/\alpha/_D^{20} = -26°5 \pm 2°$ (c = 0,7%, benzène).

*Analyse:* $C_{26}H_{27}NO_3$ PM = 401,510.
Calculé: C% = 77,78 H% =6,78 N% = 3,48
Trouvé: = 77,7 = 6,9 = 3,3

Le chlorure d'acide utilisé dans cette préparation est décrit dans le brevet français 1.505.423.

Exemple 2:

*(1R, trans) 2,2-diméthyl 3-(cyclopentylidène méthyl) cyclopropane-1-carboxylate de (S) α-cyano 3-phénoxy benzyle*

On introduit 2 g de (1R trans) 2,2-diméthyl 3-(cyclopentylidène méthyl) cyclopropane-1-carboxylate de (RS) α-cyano 3-phénoxy benzyle dont la préparation a été donnée ci-dessus dans 4 cm3 d'isopropanol et ajoute 0,2 cm3 d'ammoniaque à 22° Baumé. Après 24 heures d'agitation à 0°C, on ajoute 3 cm3 d'éther de pétrole (Eb. 60°—80°C) et agite de nouveau 48 heures à —5°C. On triture le mélange brut dans du benzène, concentre sous pression réduite, reprend par 4 cm3 d'isopropanol et 0,2 cm3 d'ammoniaque à 22° Baumé. Après 5 jours d'agitation à 5°C, on filtre puis rince à l'isopropanol glacé le produit brut cristallisé.

On dissout les cristaux dans de l'acétone, concentre sous pression réduite et obtient 1,225 g de produit attendu cristallisé, P.F ≃ 65°C $/\alpha/_D^{20} = -18° \pm 1°$ (c = 1% dans le benzène).

Le spectre de RMN pratiqué sur le produit cristallisé montre qu'il renferme environ 10% de (1R trans) 2,2-diméthyl 3-(cyclopentylidène méthyl) cyclopropane 1-carboxylate de (R) α-cyano 3-phénoxy benzyle.

Quant aux liqueurs mères, elles sont constituées par du produit de départ non transformé, le 1R trans 2,2-diméthyl 3-(cyclopentylidène méthyl) cyclopropane 1-carboxylate de (RS) α-cyano 3-phénoxy benzyle, ainsi qu'il a été établi par spectre de RMN et par $1'/\alpha/_D$ (—29° dans le benzène).

Exemple 3:

*(1R trans) 2,2-diméthyl 3-(cyclopentylidène méthyl) cyclopropane-1-carboxylate de (S) α-cyano 3-phénoxy benzyle*

On introduit 3,1 g de (1R trans) 2,2-diméthyl 3-(cyclopentylidène méthyl) cyclopropane-1-carboxylate de (RS) α-cyano 3-phénoxy benzyle dans 7 cm3 d'isopropanol, puis ajoute 0,2 ml de triéthylamine. Après 24 heures d'agitation à 0°C puis 4 jours d'agitation à —5°C, on filtre le produit obtenu, le rince à l'isopropanol glacé, le dissout dans de l'acétone et concentre sous pression réduite. On récupère 1,674 g de produit attendu cristallisé ayant des constantes identiques à celles du produit décrit dans l'exemple 2.

Exemple 4:

*(1R trans) 2,2-diméthyl 3-(cyclopentylidène méthyl cyclopropane-1-carboxylate de (S) α-cyano 3-phénoxy benzyle*

Dans 50 cm3 de benzène, on introduit 4,38 g de chlorure d'acide (1R trans) 2,2-diméthyl 3-(cyclopentylidène méthyl) cyclopropane-1-carboxylique, 4,8 g d'alcool (S) α-cyano 3-phénoxy benzylique ($/\alpha/_D^{20} = -15,5° \pm 1°$, c = 1,3%, benzène), ajoute lentement à + 5°C, 5 cm3 de pyridine, agite pendant 20 heures à 20°C, verse le mélange réactionnel dans une solution aqueuse d'acide chlorhydrique préablement refroidie à +5°C, extraite au benzène, lave la phase organique au bicarbonate de sodium, à l'eau, la sèche, la concentre à sec par distillation sous pression réduite, chromatographie le résidu sur gel de silice en éluant par un mélange d'éther de pétrole (éb = 35—70°C) et d'éther éthylique (9/1), obtient 5,08 g de produit brut (F = 70°C), recristallise ce produit dans 6 volumes d'isopropanol et recueille 4,2 g de produit attendu F = 75°C $/\alpha/_D^{20} = -11° \pm 2°$ (c =0,6%, benzène).

*Analyse*: $C_{26}H_{27}NO_3$ (401,51)
Calculé: C% 77,77 H% 6,78 N% 3,49
Trouvé: 77,8 6,8 3,3
Spectre de RMN (deutérochloroforme)

Pics à 1,13—1,20 ppm attribués aux hydrogènes des méthyles géminés substituant le cyclopropyle; pics de 1,42 à 1,51 ppm attribués aux hydrogènes en position 1 et 3 du cyclopropyle; pics de 1,50 à 1,83 ppm des hydrogènes en position 3′ et 4′ du cyclopentyle; pics de 2,00 à 2,50 ppm des hydrogènes en position 2′ et 5′ du cyclopentyle; pics à *4,95—5,08* ppm de l'hydrogène éthylénique; pics de 6,90 à 7,5 ppm attribués aux hydrogènes aromatiques.

*Dichroïsme circulaire* dioxane)
$\Delta\varepsilon = -8$ à 224 nm (maximum), $\Delta\varepsilon = +0,28$ à 283 nm (maximum), $\Delta\varepsilon = +0,3$ à 287 nm (maximum).

L'alcool (S) α-cyano 3-phénoxy benzylique utilisé au départ de l'exemple 4 peut être préparé de la manière suivante:

Stade A:

*Mélange de (1R, 5S) 6,6-diméthyl 4(R) ((S)-cyano (3'-phénoxy phényl) méthoxy) 3-oxa bicyclo (3—1—0) hexan-2-one et de (1R, 5S) 6,6-diméthyl 4(R) ((R)-cyano (3'-phénoxy phényl) méthoxy) 3-oxa bicyclo (3—1—0) hexan-2-one.*

On mélange 22,5 g d'alcool (R, S) α-cyano 3-phénoxy benzylique, 9,46 g de lactone de l'acide cis 2,2-diméthyl 3S-(dihydroxy méthyl) cyclopropan-1R-carboxylique, 0,150 g d'acide paratoluène sulfonique monohydraté, porte à 80°C sous un vide de $10^{-2}$ mm de mercure, maintient le mélange réactionnel pendant 2 heures dans ces conditions, l'eau formée étant éliminée par distillation. On refroidit à 20°C et obtient 30,70 g de mélange brut de (1R, 5S) 6,6-diméthyl 4(R) ((S)-cyano (3'-phénoxy phényl) méthoxy) 3-oxabicyclo (3—1—0) hexan-2-one et de (1R, 5S) 6,6-diméthyl 4(R) ((R)-cyano (3'-phénoxy phényl) méthoxy) 3-oxabicyclo (3—1—0) hexan-2-one (contenant comme impuretés principalement les produits de départ n'ayant pas réagi) (mélange A).

Stade B:

*(1R, 5S) 6,6-diméthyl 4(R) ((S)-cyano (3'-phénoxy phényl) méthoxy) 3-oxa bicyclo (3—1—0) hexan-2-one.*

On chromatographie le mélange A obtenu au stade A sur gel de silice, en éluant par un mélange de benzène et d'acétate d'éthyle (95/5) et obtient 10,9 g de (1R, 5S) 6,6-diméthyl 4(R) ((S)-cyano (3'-phénoxy phényl) méthoxy) 3-oxa bicyclo (3—1—0) hexan-2-one.

F = 126°C, $(\alpha)_D^{20} = -71°$ $c$ = 1%, benzène)

Spectre ultra violet (éthanol)

Inflexion à 226 nm (E$'_1$ = 319), inflexion à 267 nm (E$'_1$ = 52); inflexion à 271 nm (E$'_1$ = 56), maximum à 276 nm (E$'_1$ = 60), inflexion à 280 nm (E$'_1$ = 48).

*Dichroïsme circulaire* (dioxane)

$\Delta\varepsilon = -4{,}2$ à 225 nm (max)

$\Delta\varepsilon + 0{,}39$ à 287 nm (max).

Stade C:

*Alcool (S) α-cyano métaphénoxy benzylique.*

Dans un mélange de 100 cm3 de dioxane, de 50 cm3 d'eau, on introduit 10 g de (1R, 5S) 6,6-diméthyl 4(R) ((S) cyano (3'-phénoxy phényl) méthoxy) 3-oxabicyclo (3—1—0) hexan-2-one, obtenu au stade B ci-dessus, puis 1 g d'acide paratoluène sulfonique monohydraté, porte le mélange réactionnel au reflux, l'y maintient pendant 23 heures, concentre par distillation sous pression réduite, jusqu'à obtenir la moitié du volume initial, ajoute de l'éther éthylique, agite, sépare, par décantation la phase organique, la lave à l'eau, la sèche, la concentre à sec par distillation sous pression réduite, chromatographie le résidu (9,5 g) sur gel de silice, en éluant avec un mélange de benzène et d'acétate d'éthyle (9/1) et obtient 6,1 g d'alcool (S) α-cyano métaphénoxy benzylique $(\alpha)_D^{20} = -16{,}5° \mp 1{,}5°$ ($c$ = 0,8%, benzène).

Spectre de RMN (Deutero chloroforme)

pic à 3,25 ppm caractéristique de l'hydrogène de la fonction alcool, pic à 5,42 ppm caractéristique de l'hydrogène porté par le même carbone que le groupement nitrile.

Exemple 5:

*(1R, cis) 2,2-diméthyl 3-cyclopentylidène méthyl cyclopropane-1-carboxylate de (RS) α-cyano 3-phénoxy benzyle*

On opère de manière identique à celle décrite à l'exemple 1, au départ de 11 g d'alcool (RS) α-cyano 3-phénoxy benzylique et de 15,3 g de chlorure de l'acide (1R, cis) 2,2-diméthyl 3-cyclopentylidène méthyl cyclopropane-1-carboxylique (obtenu à partir de l'acide décrit dans le brevet français 2 076 204) et obtient après purification par chromatographie sur silice, en éluant au mélange cyclohexane — acétate d'éthyle (9—1), puis au benzène, 7,5 g de produit attendu.

$\alpha_D^{20} = +32° \pm 2°$ (c = 0,5% benzène).

*Analyse:* $C_{26}H_{27}NO_3$ = 401,507

| | C% | H% | N% |
|---|---|---|---|
| Calculé: | 77,78 | 6,78 | 3,49 |
| Trouvé: | 78,1 | 6,8 | 3,5 |

Exemple 6:

*(1R, cis) 2,2-diméthyl 3-cyclopentylidène méthyl cyclopropane-1-carboxylate de (S) α-cyano 3-phénoxy benzyle*

On opère de manière identique à celle décrite à l'exemple 5, au départ de 4,4 g de chlorure

d'acide (1R, cis) 2,2-diméthyl 3-cyclopentylidène méthyl cyclopropane-1-carboxylique et de 4,5 g d'alcool (S) $\alpha$-cyano 3-phénoxy benzylique, et obtient après purification par chromatographie sur silice en éluant au mélange benzène-cyclohexane (7—3), 1 g de produit attendu.

$\alpha_D^{20} = +85,5° \pm 3°$ (c = 0,4% benzène).

*Analyse:* $C_{26}H_{27}NO_3$ = 401,507
Calculé: C% 77,78 H% 6,78, N% 3,49
Trouvé: 78,0 6,8 3,4

Exemple 7:

*(1R, cis) 2,2-diméthyl 3-cyclobutylidène méthyl cyclopropane-1-carboxylate de (S) $\alpha$-cyano 3-phénoxy benzyle*

On opère de manière analogue à celle décrite à l'exemple 1, au départ de 2,5 g de chlorure d'acide (1R, cis) 2,2-diméthyl 3-cyclobutylidène méthyl cyclopropane-1-carboxylique (obtenu à partir de l'acide, lui-même obtenue par un procédé analogue au procédé décrit dans le brevet français 1 595 780, en faisant réagir l'aldéhyde de structure cis sur le phosphorane approprié et de 3 g d'alcool (S) $\alpha$-cyano 3-phénoxy benzylique, et obtient après purification par chromatographie sur silice en éluant au benzène, 1,9 g de produit attendu.

$\alpha_D^{20} = +95° \pm 3°$ (c = 0,54% benzène) F. = 76°C.

*Analyse:* $C_{25}H_{25}NO_3$ = 387,479
Calculé: C% 77,49 H% 6,50, N% 3,61
Trouvé: 77,2 6,3 3,5

Exemple 8:

*(1R, cis) 2,2-diméthyl 3-cyclobutylidène méthyl cyclopropane-1-carboxylate de (RS) $\alpha$-cyano 3-phénoxy benzyle*

On opère de manière analogue à celle décrite à l'exemple 1, au départ de 2,5 g de chlorure d'acide (1R, cis) 2,2-diméthyl 3-cyclobutylidène méthyl cyclopropane-1-carboxylique et de 3 g d'alcool (RS) $\alpha$-cyano 3-phénoxy benzylique, et obtient après purification par chromatographie sur silice en éluant au benzène, 2,5 g de produit attendu.

$\alpha_D^{20} = +47° \pm 0,5°$ (c = 2% benzène)

*Analyse:* $C_{25}H_{25}NO_3$ = 387,479
Calculé: C% 77,49 H% 6,50, N% 3,61
Trouvé: 77,3 6,3 3,6

Exemple 9:

*(1R, cis) 2,2-diméthyl 3-cyclobutylidène méthyl cyclopropane-1-carboxylate de (S) $\alpha$-cyano 3-phénoxy benzyle*

On introduit 2 g de (1R, cis) 2,2-diméthyl 3-cyclobutylidène méthyl cyclopropane-1-carboxylate de (RS) $\alpha$-cyano 3-phénoxy benzyle dans 8 cm3 d'isopropanol et ajoute 0,08 cm3 de triéthylamine. On amorce avec quelques cristaux de produit attendu (obtenu à l'exemple précédent), agite pendant 16 heures à 20°C et obtient, après isolement du produit, 1 g d'ester attendu cristallisé. $\alpha_D^{20} = +99,5°$ (c = 0,9% benzène). F. = 76°C.

*Analyse:* $C_{25}H_{25}NO_3$ = 387,479
Calculé: C% 77,49 H% 6,50, N% 3,61
Trouvé: 77,3 6,5 3,7

Le spectre RMN pratiqué sur le produit dans $CDCl_3$ a montré l'absence d'ester de (R) $\alpha$-cyano 3-phénoxy benzyle.

Exemple 10:

*(1R, trans) 2,2-diméthyl 3-cyclobutylidène méthyl cyclopropane-1-carboxylate de (S) $\alpha$-cyano 3-phénoxy benzyle*

On opère de manière analogue à celle décrite à l'exemple 1, au départ de 2,5 g de chlorure d'acide (1R, trans) 2-2-diméthyl 3-cyclobutylidène méthyl cyclopropane-1-carboxylique (décrit dans le certificat d'addition français n° 93 112) et de 2,2 g d'alcool (S) $\alpha$-cyano 3-phénoxy benzylique, et obtient, après purification par chromatographie sur silice en éluant au mélange éther de pétrole-éther éthylique (9,1), 1,2 g de produit attendu

$\alpha_D^{20} = -12° \pm 1°$ (c = 1% benzène).

*Analyse:* $C_{25}H_{25}NO_3$ = 387,479
Calculé: C% 77,49 H% 6,50, N% 3,61
Trouvé: 77,3 6,6 3,5

Exemple 11:

*(1R, trans) 2,2-diméthyl 3-cyclopropylidène méthyl cyclopropane-1-carboxylate de (RS) α-cyano 3-phénoxy benzyle*

On opère de manière analogue à celle décrite à l'exemple 1, au départ de chlorure d'acide (1R, trans) 2-2-diméthyl 3-cyclobutylidène méthyl cyclopropane-1-carboxylique obtenu à partir de 5,5 g de l'acide correspondant (décrit dans le certificat d'addition français n° 93 112) et de 7,5 g d'alcool (RS) α-cyano 3-phénoxy benzylique, et obtient, après purification par chromatographie sur silice en éluant au mélange benzène-éther de pétrole (8,2), 4,2 g de produit attendu.

$\alpha_D^{20}$ = −40,5 (c = 0.8% chloroforme)

*Analyse:* $C_{24}H_{23}NO_2$ = 373,428
Calculé: C% 77,19 H% 6,21, N% 3,75
Trouvé: 77,1 6,2 3,5

Exemple 12:

*(1R, trans) 2,2-diméthyl 3-cyclopropylidène méthyl cyclopropane-1-carboxylate de (RS) α-cyano 3-phénoxy benzyle*

On opère de manière analogue à celle décrite à l'exemple 1, au départ de chlorure d'acide (1R, trans) 2,2-diméthyl 3-cyclopropylidène méthyl cyclopropane-1-carboxylique obtenu à partir de 16 g d'acide correspondant, et de 27 g d'alcool (S) α-cyano 3-phénoxy benzylique, et obtient, après purification par chromatographie sur silice en éluant au mélange benzène-éther de pétrole (8,2), 16,7 g de produit attendu.

$\alpha_D^{20}$ = −38,5±1.5° (c = 1% chloroforme).

*Analyse:* $C_{24}H_{23}NO_3$ = 373,428
Calculé: C% 77,19 H% 6,21, N% 3,75
Trouvé: 77,0 6,1 3,5

Exemple 13:

*(1R, cis) 2,2-diméthyl 3-cyclopropylidène méthyl cyclopropane-1-carboxylate de (RS) α-cyano 3-phénoxy benzyle*

On opère de manière analogue à celle décrite à l'exemple 1, au départ de chlorure d'acide (1R, cis) 2-2-diméthyl 3-cyclopropylidène méthyl cyclopropane-1-carboxylique obtenu lui-même à partir de 3,5 g de acide correspondant (obtenu par un procédé analogue au procédé décrit dans le brevet français 1 595 780, en faisant réagir l'aldéhyde de structure cis sur le phosphorane approprié et de 4,8 g d'alcool (RS) α-cyano 3-phénoxy benzylique, et obtient, après purification par chromatographie sur silice en éluant au mélange benzène-éther de pétrole (8,2), 4 g de produit attendu.

$\alpha_D^{20}$ = +48°±1.5° (c = 1% chloroforme).

*Analyse:* $C_{24}H_{23}NO_3$ = 373,428
Calculé: C% 77,19 H% 6,21, N% 3,75
Trouvé: 76,7 6,2 3,7

Exemple 14:

*(1R, cis) 2,2-diméthyl 3-cyclopropylidène méthyl cyclopropane-1-carboxylate de (S) α-cyano 3-phénoxy benzyle*

On opère de manière analogue à celle décrite à l'exemple 1, au départ de chlorure d'acide (1R, cis) 2,2-diméthyl 3-cyclopropylidène méthyl cyclopropane-1-carboxylique obtenu à partir de 9 g d'acide correspondant, et de 15 g de alcool (S) α-cyano 3-phénoxy benzylique, et obtient, après purification par chromatographie sur silice en éluant au mélange benzène-éther de pétrole (8,2), 8,6 g de produit attendu.

$\alpha_D^{20}$ = +55,5° (c = 1% chloroforme).

*Analyse:* $C_{24}H_{23}NO_3$ = 373,428
Calculé: C% 77,19 H% 6,21, N% 3,75
Trouvé: 76,7 6,1 3,6

Exemple 15:

*Etude de l'activité nématicide du composé de l'exemple 1 sur Ditylenchus Myceliophagus*

Dans un pilulier contenant 10 ml de solution aqueuse à tester, on dépose 0,5 ml d'eau contenant environ 2000 nématodes. Les contrôles de mortalité sont effectués à la loupe binoculaire vingt-quatre

heures après le traitement et sur trois répétitions correspondant chacune à un prélèvement de 1 ml de la solution à tester.

Les résultats expérimentaux obtenus sont résumés dans le tableau suivant (résultats exprimés en pourcentages de mortalité) compte tenu d'un témoin non traité.

| Doses en mg de matière active par litre (ou p.p.m.) | Pourcentage de mortalité à 24 heures |
|---|---|
| 20 | 100 |
| 10 | 100 |
| 5 | 97,4 |
| 1 | 7,4 |

*Conclusion*: Le composé de l'exemple 1 est doué d'une bonne activité nématicide vis-à-vis de Ditylenchus Myceliophagus.

Exemple 16

*Etude de l'activité sur des acariens parasites des animaux des produits des exemples 1 et 5.*

1) Etude de l'activité sur larves de Boophilus Microplus:

La substance à tester est dissoute dans un mélange constitué de diméthylformamide, d'émulsifiants et d'Arcopal de façon à obtenir un concentré émulsifiable à 10%. On dilue ce concentré avec de l'eau pour obtenir des concentrations de 1000, 100 et 10 ppm.

Au moyen d'une tour à pulvérisation, on pulvérise les différentes dilutions ci-dessus sur des larves de tiques de boeufs des tropiques, de type Boophilus Microplus et on détermine après 24 heures, par comptage des larves mortes et vivantes, le pourcentage de mortalité.

Les résultats sont les suivants:

| Doses | Composé de l'exemple 1 | Composé de l'exemple 5 |
|---|---|---|
| 1000 | 100 | 100 |
| 100 | 100 | 100 |
| 10 | 75 | 100 |

2) Inhibition de la reproduction:

La substance à tester est dissoute comme au point 1) ci-dessus et on prépare les dilutions indiquées dans le tableau ci-dessous.

Des femelles adultes de Boophilus Microplus sont plongées pendant 5 minutes dans les diverses dilutions, puis sont introduites dans une enceinte chauffée, pour la ponte. On détermine

a/ le pourcentage de tiques n'ayant pas pondu,

b/ la quantité d'oeufs pondus en fonction d'un témoin,

c/ le pourcentage de larves ayant éclos.

On calcule, en fonction des chiffres obtenus, le pourcentage d'inhibition de la reproduction. 100% signifie que l'inhibition est totale et 0% que la reproduction est identique à celle obtenue avec les témoins.

Les résultats sont les suivants:

| Doses | Composé de l'exemple 1 | Composé de l'exemple 5 |
|---|---|---|
| 100 | 92 | 100 |
| 50 | 89 | 100 |
| 25 | 83 | 78 |
| 12,5 | 75 | 65 |
| 6,2 | 82 | 82 |
| 3,1 | 50 | 57 |
| 1,5 | 65 | 35 |
| 0,75 | 28 | 43 |
| 0,38 | 21 | 37 |

3) Activité systémique:

La substance à tester est dissoute dans l'huile d'olive et administrée oralement à des cobayes à la dose de 25 mg/kg. 1 heure, 3 heures et 5 heures après l'administration du produit, on laisse des punaises à jeun (Cimex Lectucarius) sucer le sang des animaux.

Dans le tableau qui suit, on a indiqué le pourcentage de mortalité après 1 heure, puis après 3 heures et enfin après 5 heures.

| | Composé de l'exemple 1 | Composé de l'exemple 5 |
|---|---|---|
| 1 h. | — | 10% |
| 3 h. | 30% | — |
| 5 h. | 30% | 20% |

Exemple 17

*Composition nématicide contenant un composé de formule I*

On a préparé un concentré émulsifiable pour traitement des sols contenant en poids 40% de 1R, trans 2,2-diméthyl 3-(cyclopentylidène méthyl) cyclopropane-1-carboxylate de (RS) $\alpha$-cyano 3-phénoxy benzyle, 6,4% d'Atlox 4851 (triglycéride oxyéthyléné combiné avec un sulfonate, indice d'acide 1,5) 3,2% d'Atlox 4855 (triglycéride oxyéthyléné combiné avec un sulfonate indice d'acide 3) et 49,6% de xylène.

Exemple 18

*Exemple de compositions pharmaceutiques:*

On a préparé des solutions répondant à la formule suivante:

| | |
|---|---|
| — (1R, trans) 2,2-diméthyl 3-cyclopentylidène méthyl cyclopropane-1-carboxylate de (RS) $\alpha$-cyano 3-phénoxy benzyle | 5 g |
| — Butoxyde de pipéronyle | 25 g |
| — Polysorbate 80 | 10 g |
| — Triton X 100 | 25 g |
| — Acétate de Tocophérol | 1 g |
| — Alcool éthylique q.s.p. | 100 ml |

On obtient ainsi une solution que l'on dilue dans 5 litres d'eau au moment de l'emploi.

Exemple 19

*Aliment composé pour animaux*

On utilise comme aliment équilibré de base, un aliment comportant du maïs, de la luzerne déshydratée, de la paille de blé, du tourteau de palmiste mélassé, de l'urée, un condiment minéral vitaminique.

Cet aliment contient au minimum 11% de matières protéiques brutes (dont 2,8% apporté par l'urée), 2,5% de matières grasses et au maximum 15% de matières cellulosiques, 6% de matières minérales et 13% d'humidité.

L'aliment utilisé correspond à 82 unités fourragères pour 100 kilos et contient, pour 100 kilos : 910.000 U.I. de vitamine A, 91.000 U.I. de vitamine $D_3$, 156 mg de vitamine E et 150 mg de vitamine C.

On incorpore à cet aliment, 0,04 kg de (1R, trans) 2,2-diméthyl 3-cyclopentylidène méthyl cyclopropane-1-carboxylate de (RS) $\alpha$-cyano 3-phénoxy benzyle.

**Revendications**

1. Les composés de formule ($I_A$):

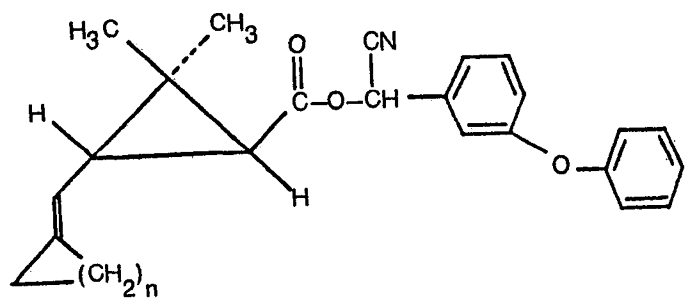

($I_A$)

dans laquelle n est un entier égal à 1, 2 ou 3, la configuration de la copule acide est (1R, trans) ou (1R, cis) et celle de la copule alcoolique est (S).

2. A titre de composé tel que défini à la revendication 1 le 1R, trans 2,2-diméthyl 3-(cyclopentylidène méthyl) cyclopropane-1-carboxylate de (S) $\alpha$-cyano 3-phénoxy benzyle.

3. A titre de composé tel que défini à la revendication 1 le 1R, cis 2,2-diméthyl 3-(cyclopentylidène méthyl) cyclopropane-1-carboxylate de (S) $\alpha$-cyano 3-phénoxy benzyle.

4. A titre de composé tel que défini à la revendication 1 le 1R, cis 2,2-diméthyl 3-(cyclobutylidène méthyl) cyclopropane-1-carboxylate de (S) $\alpha$-cyano 3-phénoxy benzyle.

5. A titre de composé tel que défini à la revendication 1 le 1R, trans 2,2-diméthyl 3-(cyclobutylidène méthyl) cyclopropane-1-carboxylate de (S) $\alpha$-cyano 3-phénoxy benzyle.

6. A titre de composé tel que défini à la revendication 1 le 1R, cis 2,2-diméthyl 3-(cyclopropylidène méthyl) cyclopropane-1-carboxylate de (S) $\alpha$-cyano 3-phénoxy benzyle.

7. A titre de composé tel que défini à la revendication 1 le 1R, trans 2,2-diméthyl 3-(cyclopropylidène méthyl) cyclopropane-1-carboxylate de (S) $\alpha$-cyano 3-phénoxy benzyle.

8. Procédé de préparation des produits de formule $I_A$ telle que définie à la revendication 1, caractérisé en ce que l'on fait réagir un acide de formule II de structure (1R, cis) ou (1R, trans):

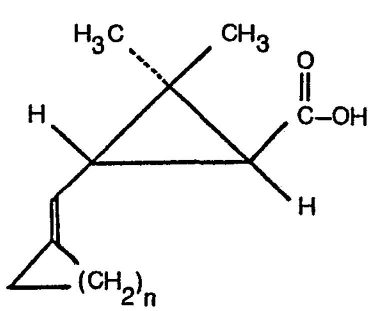

(II)

dans lequel n est défini comme à la revendication 1, ou l'un de ses dérivés fonctionnels, avec l'alcool (S) $\alpha$-cyano 3-phénoxy benzylique ou avec l'alcool (RS) $\alpha$-cyano 3-phénoxy benzylique, pour obtenir l'ester

correspondant de (S) ou de (RS) α-cyano 3-phénoxy benzyle, et soumet, le case échéant, l'ester de (RS) α-cyano 3-phénoxy benzyle à l'action d'un agent basique au sein d'un solvant dans lequel l'ester correspondant d'alcool de configuration (S) est insoluble et dans lequel l'ester correspondant d'alcool de configuration (R) est soluble, pour obtenir l'ester de (S) α-cyano 3-phénoxy benzyle.

9. Procédé selon la revendication 8, caractérisé en ce que l'on soumet l'ester de (RS) α-cyano 3-phénoxy benzyle à l'action d'un agent basique, au sein d'un solvant dans lequel l'ester correspondant d'alcool de configuration (S) est insoluble et dans lequel l'ester correspondant d'alcool de configuration (R) est soluble, et obtient l'ester de (S) α-cyano 3-phénoxy benzyle.

10. Procédé selon les revendications 8 ou 9, caractérisé en ce que l'agent basique utilisé pour la transformation de l'ester d'alcool (RS) en ester d'alcool (S) est choisi dans le groupe constitué par l'ammoniaque, la triéthylamine, la diéthylamine, la morpholine, la pyrrolidine, la pipéridine, et utilisés en quantité catalytique, la soude, la potasse, les alcoolates alcalins, les amidures alcalins, les hydrures alcalins, et en ce que le solvant ou le mélange de solvants utilisés pour insolubiliser l'ester d'alcool (S) est choisi dans le groupe constitué par l'acéto nitrile, les alcanols et les mélanges d'alcanol et d'éther de pétrole, notamment les mélanges d'alcanol et de pentane, d'hexane ou d'heptane.

11. Compositions nématicides, caractérisées en ce qu'elles renferment, comme principe actif, l'un au moins des composés de formula I:

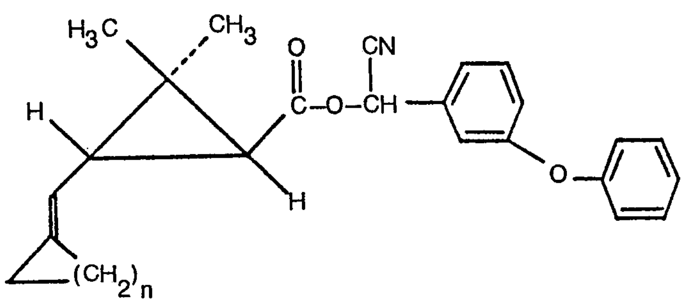

(I)

dans laquelle n est un entier égal à 1, 2 ou 3, la configuration de la copule acide est (1R, trans) ou (1R, cis) et celle de la copule alcoolique est (RS) ou (S).

12. A titre de médicaments vétérinaires, destinés à la lutte contre les acariens parasites des animaux, les composés de formule I, tels que définis à la revendication 11.

13. Compositions renfermant, à titre de principe actif, l'un au moins des produits de formule I, tels que définis à la revendication 11, et destinées à la lutte contre les acariens parasites des animaux.

14. Compositions pharmaceutiques vétérinaires, renfermant à titre de principe actif, l'un au moins des produits de formule I tels que définis à la revendication 11 et destinées à la lutte contre les acariens parasites des animaux.

15. Compositions destinées à l'alimentation animale, caractérisées en ce qu'elles sont constituées par un aliment composé équilibré pour animal et qu'elles renferment, en outre, un au moins des composés de formule I, tels que définis à la revendication 11.

## Claims

1. The compounds with the formula ($I_A$):

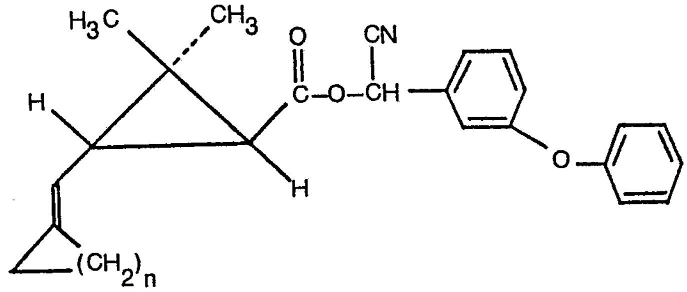

($I_A$)

in which n is an integer equal to 1, 2 or 3, the configuration of the acid copula is (1R, trans) or (1R, cis) and that of the alcohol copula is (S).

2. As compound as defined in claim 1, 1R, trans 2,2-dimethyl 3-(cyclopentylidene methyl) cyclopropane-1-carboxylate of (S) α-cyano 3-phenoxy benzyl.

3. As compound as defined in claim 1, 1R. cis 2.2-dimethyl 3-(cylcopentylidene methyl) cyclopropane-1-carboxylate of (S) α-cyano 3-phenoxy benzyl.

4. As compound as defined in claim 1, 1R, cis 2.2-dimethyl 3-(cyclobutylidene methyl) cyclopropane-1-carboxylate of (S) α-cyano 3-phenoxy benzyl.

5. As compound as defined in claim 1, 1R, trans 2.2-dimethyl 3-(cyclobutylidene methyl) cyclopropane-1-carboxylate of (S) α-cyano 3-phenoxy benzyl.

6. As compound as defined in claim 1, 1R, cis 2.2-dimethyl 3-(cyclopropylidene methyl) cyclopropane-1-carboxylate of (S) α-cyano 3-phenoxy benzyl.

7. As compound as defined in claim 1, 1R, trans 2,2-dimethyl 3-(cyclopropylidene methyl) cyclopropane-1-carboxylate of (S) α-cyano 3-phenoxy benzyl.

8. Process for the preparation of the products with the formula $I_A$, as defined in claim 1, characterized in that an acid with the formula II and with the structure (1R, cis) or (1R, trans):

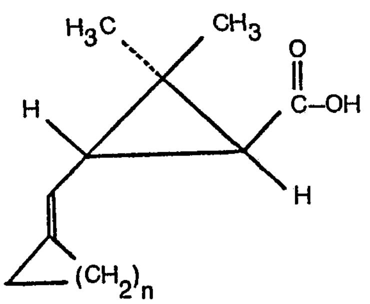

(II)

in which n is defined as in claim 1, or one of its functional derivatives, is made to react with (S) α-cyano 3-phenoxy benzyl alcohol or with (RS) α-cyano 3-phenoxy benzyl alcohol so as to obtain the corresponding ester of (S) or of (RS) α-cyano 3-phenoxy benzyl, and, if required, the ester of (RS) α-cyano 3-phenoxy benzyl is submitted to the action of a basic agent in a solvent in which the corresponding ester of (S) configuration alcohol is insoluble and in which the corresponding ester of (R) configuration alcohol is soluble, and obtain the ester of (S) α-cyano 3-phenoxy benzyl.

9. Process according to claim 8, characterized in that the ester of (RS) α-cyano 3-phenoxy benzyl is submitted to the action of a basic agent in a solvent in which the corresponding ester of (S) configuration alcohol is insoluble and in which the corresponding ester of (R) configuration alcohol is soluble, and obtain the ester of (S) α-cyano 3-phenoxy benzyl.

10. Process according to the claims 8 or 9, characterized in that the basic agent utilized for the transformation of the ester of (RS) alcohol into the ester of (S) alcohol is chosen from the group constituted by ammonia, triethylamine, diethylamine, morpholine, pyrrolidine, piperidine, and utilized in catalytic quantities, sodium hydroxide, potassium hydroxide, the alkaline alcoholates, the alkaline amides, the alkaline hydrides, and in that the solvent or the mixture of solvents utilized to make the ester of (S) alcohol insoluble is chosen form the group constituted by aceto nitrile, the alkanols and the mixtures of alkanol and petroleum ether, in particular the mixtures of alkanol and pentane, hexane or heptane.

11. Nematocidal compositions, characterized in that they contain, as active principle, one at least of the compounds with the formula I:

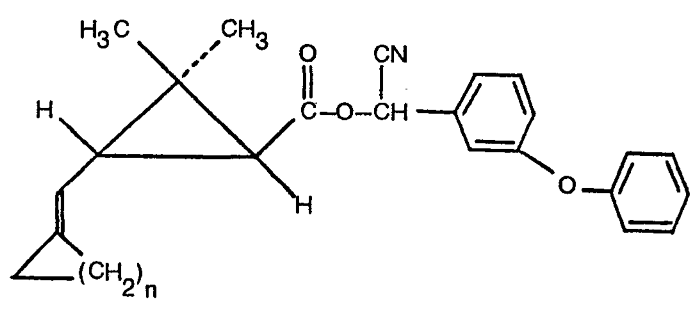

(I)

in which n is an integer equal to 1, 2 or 3, the configuration of the acid copula is (1R, trans) or (1R, cis) and that of the alcohol copula is (RS) or (S).

12. As veterinary medicaments, intended to combat parasitic acaridae of animals, the compounds with the formula I, as defined in claim 11.

13. Compositions containing, as active principle, one at least of the products with the formula I, as defined in claim 11, and intended for combatting the parasitic acaridae of animals.

14. Pharmaceutical veterinary compositions, containing as active principle at least one of the products with the formula I as defined in claim 11 and intended for combatting the parasitic acaridae of animals.

15. Compositions intended for animal alimentation, characterized in that they are constituted by a balanced compound aliment for animal and that they contain, in addition, one at least of the compounds with the formula I, as defined in claim 11.

## Patentansprüche

1. Verbindungen der Formel $I_A$

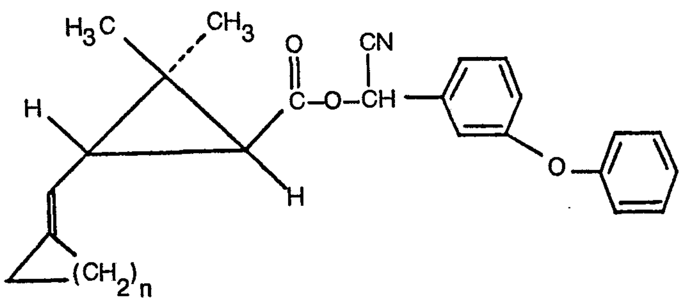

($I_A$)

worin n eine ganze Zahl entsprechend 1, 2 oder 3 ist, die Konfiguration der Säureverknüpfung (1R, trans) oder (1R, cis) ist und diejenige der Alkoholverknüpfung (S) ist.

2. Als Verbindung gemäß Anspruch 1 (S)-$\alpha$-Cyano-3-phenoxybenzyl-1R, trans-2,2-dimethyl-3-(cyclopentylidenmethyl)-cyclopropan-1-carboxylat.

3. Als Verbindung gemäß Anspruch 1 (S)-$\alpha$-Cyano-3-phenoxybenzyl-1R, cis-2,2-dimethyl-3-(cyclopentylidenmethyl)-cyclopropan-1-carboxylat.

4. Als Verbindung gemäß Anspruch 1 (S)-$\alpha$-Cyano-3-phenoxybenzyl-1R, cis-2,2-dimethyl-3-(cyclobutylidenmethyl)-cyclopropan-1-carboxylat.

5. Als Verbindung gemäß Anspruch 1 (S)-$\alpha$-Cyano-3-phenoxybenzyl-1R, trans-2,2-dimethyl-3-(cyclobutylidenmethyl)-cyclopropan-1-carboxylat.

6. Als Verbindung gemäß Anspruch 1 (S)-$\alpha$-Cyano-3-phenoxybenzyl-1R, cis-2,2-dimethyl-3-(cyclopropylidenmethyl)-cyclopropan-1-carboxylat.

7. Als Verbindung gemäß Anspruch 1 (S)-$\alpha$-Cyano-3-phenoxybenzyl-1R, trans-2,2-dimethyl-3-(cyclopropylidenmethyl)-cyclopropan-1-carboxylat.

8. Verfahren zur Herstellung der Produkte der Formel $I_A$ gemäß Anspruch 1, dadurch gekennzeichnet, daß man eine Säure der Formel II mit (1R, cis)- oder (1R, trans)-Struktur

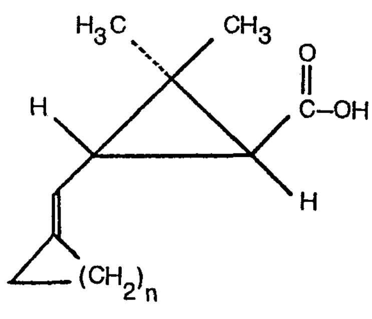

(II)

worin n wie in Anspruch 1 definiert ist, oder eines ihrer funktionellen Derivate mit dem (S)-$\alpha$-Cyano-3-phenoxybenzylalkohol oder mit dem (RS)-$\alpha$-Cyano-3-phenoxybenzylalkohol umsetzt, um den entsprechenden (S)- oder (RS)-$\alpha$-Cyano-3-phenoxybenzylester zu erhalten und gegebenenfalls den (RS)-$\alpha$-Cyano-3-phenoxybenzylester der Einwirkung eines basischen Mittels in dem Medium eines Lösungsmittels, in dem der entsprechende Ester des Alkohols mit (S)-Konfiguration unlöslich ist und in dem der entsprechende Ester des Alkohols mit (R)-Konfiguration löslich ist, unterzieht, um den (S)-$\alpha$-Cyano-3-phenoxybenzylester zu erhalten.

9. Verfahren gemäß Anspruch 8, dadurch gekennzeichnet, daß man den Ester des (RS)-$\alpha$-Cyano-3-phenoxybenzylalkohols der Einwirkung eines basischen Mittels in dem Medium eines Lösungsmittels, in dem der entsprechende Ester des Alkohols mit (S)-Konfiguration unlöslich ist und in dem der entsprechende Ester des Alkohols mit (R)-Konfiguration löslich ist, unterzieht und den (S)-$\alpha$-Cyano-3-phenoxybenzylester erhält.

10. Verfahren gemäß Anspruch 8 oder 9, dadurch gekennzeichnet, daß das für die Überführung des Esters des (RS)-Alkohols in den Ester des (S)-Alkohols verwendete basische Mittel ausgewählt wird unter Ammoniak, Triäthylamin, Diäthylamin, Morpholin, Pyrrolidin, Piperidin, und in katalytischer Menge verwendet, Natronlauge, Kalilauge, den Alkalialkoholaten, den Alkaliamiden, den Alkalihydriden, und daß das Lösungsmittel oder das Gemisch der Lösungsmittel, die für die Unlöslichmachung des Esters des (S)-Alkohols verwendet werden, ausgewählt werden unter Acetonitril, den Alkanolen und Gemischen von Alkanol und Petroläther, insbesondere Gemischen von Alkanol und Pentan, Hexan oder Heptan.

11. Nematizide Zusammensetzungen, dadurch gekennzeichnet, daß sie als Wirkstoff zumindest eine der Verbindungen der Formel I

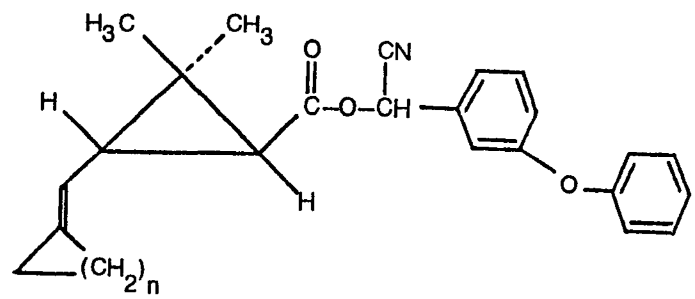

(I)

enthalten, worin n die ganze Zahl 1, 2 oder 3 ist, die Konfiguration der Säureverknüpfung (1R, trans) oder (1R, cis) ist und diejenige der Alkoholverknüpfung (RS) oder (S) ist.

12. Als veterinäre Arzneimittel für die Bekämpfung von parasitären Milben der Tiere die Verbindungen der Formel I gemäß Anspruch 11.

13. Zusammensetzungen, enthaltend als Wirkstoff zumindest eines der Produkte der Formel I gemäß Anspruch 11 und bestimmt für die Bekämpfung von parasitären Milben der Tiere.

14. Veterinäre pharmazeutische Zusammensetzungen, enthaltend als Wirkstoff zumindest eines der Produkte der Formel I gemäß Anspruch 11 und bestimmt für die Bekämpfung von parasitären Milben der Tiere.

15. Zusammensetzungen für die tierische Ernährung, dadurch gekennzeichnet, daß sie aus einer dem Tier angepaßten Nahrungsmittelzusammensetzung bestehen und außerdem zumindest eine der Verbindungen der Formel I gemäß Anspruch 11 enthalten.